(19) Europäisches Patentamt
European Patent Office
Office européen des brevets

(11) **EP 4 390 401 A1**

(12) **EUROPEAN PATENT APPLICATION**
published in accordance with Art. 153(4) EPC

(43) Date of publication:
**26.06.2024 Bulletin 2024/26**

(21) Application number: **22857083.4**

(22) Date of filing: **19.07.2022**

(51) International Patent Classification (IPC):
**G01N 35/08** (2006.01)

(52) Cooperative Patent Classification (CPC):
**G01N 35/08**

(86) International application number:
**PCT/JP2022/027987**

(87) International publication number:
**WO 2023/021904 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.08.2021 JP 2021135063**

(71) Applicant: **HITACHI HIGH-TECH CORPORATION**
**Tokyo 105-6409 (JP)**

(72) Inventors:
• **ITO, Hiroshi**
  **Tokyo 100-8280 (JP)**
• **FUKUMURA, Mami**
  **Tokyo 100-8280 (JP)**
• **SUGIMURA, Yoshiaki**
  **Tokyo 105-6409 (JP)**
• **OGUCHI, So**
  **Tokyo 105-6409 (JP)**

(74) Representative: **MERH-IP Matias Erny Reichl Hoffmann**
**Patentanwälte PartG mbB**
**Paul-Heyse-Strasse 29**
**80336 München (DE)**

(54) **SAMPLE ANALYZER**

(57) Provided is a sample analyzer that achieves an increase in the capturing rate of magnetic particles. A sample analyzer includes a channel configured to introduce a sample liquid containing magnetic particles bound to a specific substance into a capturing region, a supply unit configured to supply the sample liquid to the channel, a capturing unit having a magnetic field structure for generating a magnetic field and configured to adsorb the magnetic particles to the capturing region by the magnetic field, a measurement unit configured to measure the specific substance captured to the capturing region, and a discharge unit configured to discharge the magnetic particles from the channel after measurement by the measurement unit. The magnetic field structure includes a plurality of magnets arranged outside the channel and having different magnetization directions, and the plurality of magnets are arranged so as to make the magnetic flux density on a channel side larger than that on a side facing the channel.

FIG. 4

EP 4 390 401 A1

## Description

Technical Field

The present invention relates to a sample analyzer, for example, a device using a reaction between an antigen and an antibody.

Background Art

First, immunoassay will be described as an example of sample analysis.

An immunological test is to detect or measure an antibody or an antigen in a body fluid (plasma, serum, urine, and the like) using a specific reaction between an antigen and an antibody for the purpose of diagnosis of a disease or diagnosis of a disease state. As a representative method, there is an ELISA method (Enzyme-Linked Immunosorbent Assay).

As a typical execution process of the ELISA method, an antibody (first antibody) against an antigen to be measured is immobilized in a vessel, a sample such as plasma, serum, or urine is placed therein, and the antigen in the sample is bound to the first antibody. In addition, an antibody (second antibody) to which a label is bound is further bound to the antigen bound to the first antibody. A conjugate of the first antibody, the antigen, the second antibody, and the label is collected, and a signal emitted from the label is detected to measure the presence or absence and amount of antigen in the sample.

As a label, for example, a fluorescent substance or the like is used. In this case, the luminescence increases in proportion to the number of the second antibodies to which the label is bound, that is, the amount of antigen in the conjugate, and the luminescence of the fluorescent substance is detected with a photomultiplier tube or the like, whereby the antigen in the sample can be quantified.

In a specific example of an immunological test apparatus using the ELISA method, magnetic particles are used as a solid phase, and the first antibody is immobilized on the surfaces of the magnetic particles. A substance (fluorescent labeling substance) to which a fluorescent dye is bound as a label is bound to the second antibody. By mixing a biologically derived detection substance (antigen) and a magnetic particle on which the first antibody is immobilized and causing an antigen-antibody reaction, a specific antigen contained in the sample is bound to the magnetic particle via the first antibody. Furthermore, when the second antibody is reacted, the fluorescent labeling substance binds to the magnetic particles via the second antibody, the antigen, and the first antibody. The amount of fluorescent labeling substance increases or decreases depending on the amount of detection substance contained in the sample, that is, the amount of antigen.

While causing the sample containing magnetic particles to which the detection substance is bound to flow through an arbitrary channel, the magnetic particles are captured at a target position in the middle of the channel. By causing a laser or the like to act on the captured magnetic particles, the fluorescent labeling substance bound to the magnetic particles is caused to emit light. By detecting the emission intensity at this time, the amount of detection substance in the sample, that is, the amount of antigen can be measured, thus performing quantitative measurement.

In order to perform highly sensitive immunoassay, so-called B/F separation (Bound/Free separation or separation of antigen-antibody conjugates and non-conjugates) is performed in which magnetic particles to which a detection substance (antigen) is bound are captured and trapped at a specific location using a magnet or the like, and a solution containing an antibody not bound to an antigen is replaced.

PTL 1 discloses a method of capturing magnetic particles at a target position using a magnet or the like in an analyzer.

In addition, NPL 1 describes, for example, an action of a magnetic field on a substance.

Citation List

Patent Literature

PTL 1: WO 2011/ 155489A

Non Patent Literature

NPL 1: Motokawa, "High Magnetic Field and Material Science", Material Japan, Vol. 37, (1998), pp. 926-930

Summary of Invention

Technical Problem

When the magnetic particles are captured using a magnet or the like, it is necessary to perform adsorption to a target range (target position) on a channel wall surface in order to efficiently perform light emission and detection of emission intensity. However, the adsorption of magnetic particles to the target position has the following problems.

In order to capture magnetic particles at a target position, a magnetic field is generated using a magnet or the like, and a magnetic force is applied to the magnetic particles. However, when there is a portion with a weak magnetic field within the range of the target position, magnetic particles cannot be sufficiently captured. That is, the capturing rate of magnetic particles is reduced. This is particularly remarkable when, for example, the flow velocity of the sample liquid in the channel is large or when the cross-sectional area of the channel is large, and the magnetic field of the magnet hardly affects the entire channel cross-section. Here, the capturing rate is the ratio of magnetic particles that can be collected at a target position among the magnetic particles contained in the sample.

Even when the magnetic field is sufficiently strengthened within the range of the target position, the behavior of magnetic particles becomes asymmetric on the upstream side and the downstream side according to the orientation of flow in the process of capturing magnetic particles from the fluid. In addition, since the fluid behavior is different between the near the wall and near the center in the channel, the behavior of magnetic particles is different. From these, the adsorption distribution of magnetic particles may be uneven.

With respect to the uneven adsorption distribution of magnetic particles, for example, the magnetic particles captured at a position out of the target position do not contribute to light emission, and hence measurement performance deteriorates. In addition, in the inside of the target position, the density of captured magnetic particles may vary, and the light emission sensitivity decreases in a sparse portion, so that the measurement performance also deteriorates in this case.

In addition, at the occurrence of adsorption of magnetic particles out of a target position or variation in density at the time of adsorption, when the solution is replaced at the time of B/F separation, the solution remains in the aggregated portion of magnetic particles due to the interfacial tension of the solution, and sufficient B/F separation cannot be achieved.

PTL 1 discloses a structure in which a magnet is disposed in an inclined manner and a structure in which an end portion of the magnet is cut off as a method of preventing uneven adsorption due to upstream/downstream asymmetry. Such a method in which the distance between the channel and the magnet is increased on the upstream side to weaken the magnetic field achieves even collection of magnetic particles but partially impairs the original material function of the magnet and the superiority of the magnet arrangement. Accordingly, an increase in the capturing rate of magnetic particles has been a problem. In this case, the material function is, for example, the residual magnetic flux density of the material, and the superiority of the magnet arrangement is, for example, that the magnet is at a position close to a target for which a magnetic field is desired to be generated.

In view of the above problems, an object of the present invention is to provide a sample analyzer that achieves an increase in the capturing rate of magnetic particles.

Solution to Problem

An example of a sample analyzer according to the present invention includes

    a channel configured to introduce a sample liquid containing magnetic particles bound to a specific substance into a capturing region,
a supply unit configured to supply the sample liquid to the channel,
a capturing unit having a magnetic field structure for generating a magnetic field and configured to adsorb the magnetic particles to the capturing region by the magnetic field,
a measurement unit configured to measure the specific substance captured to the capturing region, and
a discharge unit configured to discharge the magnetic particles from the channel after measurement by the measurement unit.

The magnetic field structure includes a plurality of magnets arranged outside the channel and having different magnetization directions. The plurality of magnets are arranged so as to make a magnetic flux density on a channel side larger than a magnetic flux density on a side opposite to the channel.

The present specification includes the disclosure of Japanese Patent Application No. 2021-135063 on which priority of the present application is based.

Advantageous Effects of Invention

The sample analyzer according to the present invention can increase the capturing rate of magnetic particles.

Brief Description of Drawings

    [FIG. 1] FIG. 1 is a schematic configuration diagram of an immunoassay apparatus according to a first embodiment.
[FIG. 2] FIG. 2 is a schematic configuration diagram of an immunoassay device using laser light.
[FIG. 3] FIG. 3 is an enlarged view of a configuration near the channel of the immunoassay apparatus according to the first embodiment.
[FIG. 4] FIG. 4 is an enlarged view near the channel according to the first embodiment.
[FIG. 5] FIG. 5 is a diagram of a magnet structure and generated magnetic flux according to the first embodiment.
[FIG. 6] FIG. 6 shows magnetic field analysis results according to conventional art and the first embodiment.
[FIG. 7] FIG. 7 shows a magnetic field analysis result of the upper surface of the magnet according to conventional art and the first embodiment.
[FIG. 8] FIG. 8 is an analysis flowchart for analyzing the behavior of magnetic particles according to the first embodiment.
[FIG. 9] FIG. 9 shows the behavior analysis results of magnetic particles when the magnet structures according to conventional art and the first embodiment are used.

[FIG. 10] FIG. 10 shows the histograms of particle positions in the behavior analysis of magnetic particles according to conventional art and the first embodiment.

[FIG. 11] FIG. 11 shows a magnet structure according to a second embodiment.

[FIG. 12] FIG. 12 shows a magnet structure according to a modification in which the number of magnets in the second embodiment is changed.

[FIG. 13] FIG. 13 shows a magnet structure according to a third embodiment.

[FIG. 14] FIG. 14 shows a magnet structure according to a fourth embodiment.

[FIG. 15] FIG. 15 shows a magnet structure according to a fifth embodiment.

Description of Embodiments

Hereinafter, embodiments of the present invention will be described with reference to the accompanying drawings.

First Embodiment

As an example of a sample analyzer according to the present embodiment, a certain immunoassay apparatus will be described. The present invention is not limited to immunoassay, can be applied to any sample analyzer that traps magnetic particles using magnetic particles, and can be similarly used for analyzers such as DNA and biochemistry analyzers. In addition, in the following description, unless otherwise specified, with regard to the terms "direction" and "orientation", assume that "direction" simply means a linear state, and "orientation" means movement from a certain start point toward one side according to general physical expressions.

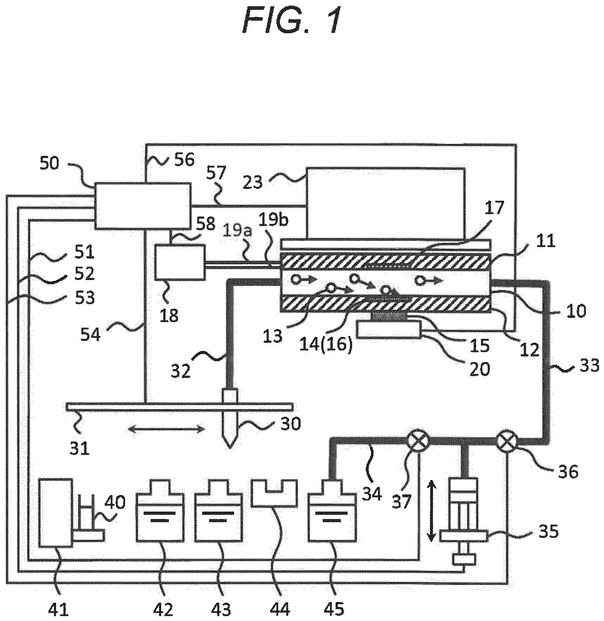

FIG. 1 is a schematic configuration diagram of an immunoassay apparatus. Referring to FIG. 1, a channel 10 is connected to a sipper nozzle 30 and a pump 35 through a tube 32 and a tube 33. The sipper nozzle 30 is movably attached by an arm 31, and a suspension vessel 40, a cleaning liquid vessel 42, and a buffer solution vessel 43 are installed in a movement range of the sipper nozzle.

A valve 36 is provided in the tube 33 between the channel 10 and the pump 35. The pump 35 is controlled by a controller 50 through a signal line 52 and can aspirate and discharge accurate liquid amounts. The pump 35 further continues to a waste liquid vessel 45 through a tube 34.

A portion constituted by the channel 10, channel walls 11 and 12, and a target adsorption position 14 is referred to as a detection unit (alternatively, a flow cell as an integrated member). The channel wall 11 of the flow cell is formed of a transparent material, and the channel 10 through which a solution flows is formed therein. Each of the channel walls 11 and 12 can be configured using, for example, a planar plate but is not limited thereto. Since the channel wall 11 is formed of a transparent material, light is transmitted, and an internal flow state can be observed. The entire channel wall 11 is not necessarily transparent, and only a portion through which light is transmitted may be transparent as a window.

The channel wall 11 is preferably made of a material that is substantially transparent to the wavelength of light emitted by the labeling substance of the magnetic particle conjugate captured to the adsorption portion in the flow cell and is preferably made of, for example, glass or plastic.

A reaction field electrode 16 is installed at the target adsorption position 14 installed in the channel 10. In addition, a counter electrode 17 is installed from the reaction field electrode 16 (target adsorption position 14) to a facing circumference in the channel 10. Further, the reaction field electrode 16 and the counter electrode 17 are connected to a voltage applying unit 18 via lead wires 19a and 19b. The voltage applying unit 18 is connected to the controller 50 via a signal line 58.

Furthermore, in order to capture magnetic particles, a magnet 15 is used as a magnetic field application unit. The magnet 15 is a permanent magnet or an electromagnet. When magnetic particles are to be captured, for example, the magnet 15 is moved to immediately below the channel 10. For example, the magnet 15 is installed on a slide mechanism 20 that can be freely moved in the horizontal direction, and the magnet 15 is moved immediately below the channel 10 when the magnetic particles are to be captured.

The magnetic particles 13 are captured at the target adsorption position 14 in the channel 10 by the magnetic force received from the magnetic field of the magnet 15. When the target adsorption position 14 is a surface having a target area, light emission measurement is easy to perform. In the present embodiment, the target adsorption position 14 is provided on the bottom surface of the channel 10 but may be provided on another surface in the channel 10 or on a plurality of three-dimensionally arranged surfaces.

When the inside of the channel 10 is to be cleaned, for example, the magnet 15 is moved to a position where the influence of the magnet 15 in the channel 10 can be sufficiently reduced, whereby the inside of the channel can be sufficiently cleaned.

The slide mechanism 20 is not necessarily moved in the horizontal direction and may be moved in the vertical direction or in both the horizontal and vertical directions as long as the influence of the magnetic field during cleaning can be reduced. In addition, the magnet 15 may be an electromagnet. In this case, the magnetic field in the channel 10 can be controlled not by movement of the position but by an applied current.

The controller 50 is connected to the valve 36, a valve 37, the pump 35, the arm 31, the voltage applying unit 18, a photodetector 23, and the slide mechanism 20 by a signal line 51, the signal line 52, signal lines 53, 54, 56, and 57, and the signal line 58 so that they can be independently controlled.

In measurement, the controller 50 controls the voltage applying unit 18. Accordingly, when a voltage is applied between the reaction field electrode 16 and the counter electrode 17 in the channel 10, light can be electrochemically emitted from the labeling substance bound to the magnetic particles 13 captured on the reaction field electrode 16 (target adsorption position 14). As long as the portion of the channel wall 11 which surrounds the counter electrode 17 is made of a transparent material so as to enable measurement by the photodetector 23, the remaining portion need not be made of a transparent material.

The materials of the reaction field electrode 16 and the counter electrode 17 can include, for example, gold, platinum, palladium, tungsten, iridium, nickel, alloys thereof, and carbon materials. In addition, as each of the reaction field electrode 16 and the counter electrode 17, for example, a film formed by plating, sputtering, and the like of the above material on a base material such as titanium can also be used.

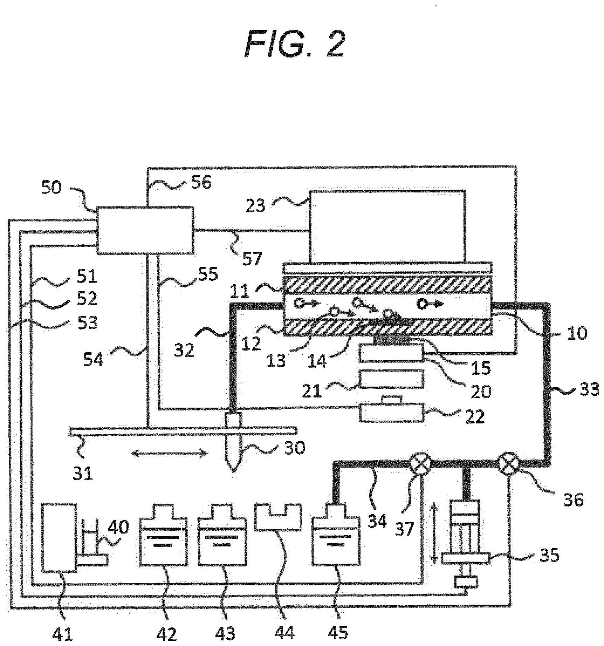

By adopting the light-emitting method using the counter electrode 17, the reaction field electrode 16 and the counter electrode 17 can be fixed to the flow cell. Since the immunoassay apparatus in FIG. 1 does not require a condenser lens 21 and a laser light source 22 unlike the immunoassay apparatus using laser light described later with reference to FIG. 2, positioning of laser light and selection and installation of a transparent material at the target adsorption position 14 can be omitted, and hence simplification of the apparatus and suppression of variations in light emission can be expected.

As the photodetector 23, for example, a camera or a photomultiplier tube can be used.

The sample to be analyzed is a substance derived from a living body such as serum or urine. When the sample is serum, the specific component to be analyzed is, for example, a tumor marker, an antibody, an antigen-antibody conjugate, or a single protein. In the following description, assume that a specific component is TSH (thyroid stimulating hormone).

The suspension vessel 40 contains the liquid (suspension) obtained by mixing a sample to be analyzed with a bead solution and a reagent, as a pretreatment process, and then making the mixture be reacted at a constant temperature (for example, 37°) for a certain period of time. The bead solution is a solution in which magnetic particles 13 having a particulate magnetic substance embedded in a matrix material such as polystyrene are dispersed in a buffer solution, and streptavidin capable of binding to biotin is bound to the surface of the matrix material. A reagent contains a substance that binds the magnetic particles 13 to the specific component TSH in the sample and includes an anti-TSH (Thyroid Stimulating Hormone) antibody whose terminal is treated with biotin. Reagents vary depending on the type of particular component to be analyzed, for example, immunoglobulins, antigens, antibodies or other biological substances are used.

The cleaning liquid vessel 42 contains a cleaning liquid for cleaning the insides of the channel 10 and the tube 32.

When the length of the channel 10 in a direction along the flow (path length), the thickness (vertical dimension) of a cross-section perpendicular to the flow, and the width (horizontal dimension) are defined, the shape of the channel 10 is preferably formed such that the path length is 2 to 20 times the larger one of the thickness and the width. This is because by sufficiently securing the path, the magnetic particles 13 in the fluid spread in the channel 10 and then are easily captured to the target adsorption position 14 provided on the bottom surface of the channel 10.

The adsorption distribution of the magnetic particles 13 in the channel 10 is determined by the magnetic force received from the magnet 15 installed near the channel 10 and the drag due to the flow of the fluid (suspension). The magnetic field in the channel 10 preferably has a magnitude of a magnetic flux density of 0.1T to 0.5T. The flow velocity of the fluid in that case is preferably 0.05 m/s to 0.10 m/s.

The particles used as the magnetic particles 13 are preferably particles as described below: (1) particles exhibiting paramagnetic, superparamagnetic, ferromagnetic, or ferrimagnetic and (2) particles obtained by embedding particles exhibiting paramagnetic, superparamagnetic, ferromagnetic, or ferrimagnetic in a material such as a synthetic polymer compound (polystyrene, nylon, or the like), a natural polymer (cellulose, agarose, or the like), or an inorganic compound (silica or the like).

The particle size is preferably in the range of 0.01 $\mu$m to 200 $\mu$m, and more preferably in the range of 1 $\mu$m to 10 $\mu$m. The specific gravity is preferably 1.3 to 1.5. With this specification, the magnetic particles 13 hardly settle in the liquid and are easily suspended. A substance having a property of specifically binding the substance to be analyzed, for example, an antibody having a property of specifically binding to an antigen is bound to the surface of a particle.

The labeling substance is preferably a substance as described below. Specifically, the following examples are given from the viewpoint of specifically binding the labeling substance to the substance to be analyzed by an appropriate means and emitting light by an appropriate means.

(1) A labeling substance used in fluorescence immunoassay. For example, an antibody labeled with fluorescein isothiocyanate.

(2) A labeling substance used in chemiluminescent immunoassay. For example, an antibody labeled with acridinium ester.

(3) A labeling substance used in chemiluminescent enzyme immunoassay. For example, an antibody labeled with a chemiluminescent enzyme using luminol or an adamantyl derivative as a luminescent substrate.

The above is an example of the immunoassay apparatus (FIG. 1) according to the present embodiment. However, as an example of an apparatus according to a modification having a different mechanism, FIG. 2 shows a schematic configuration diagram of an immunoassay apparatus using laser light.

Referring to FIG. 2, the laser light source 22 and the condenser lens 21 are installed around under the channel 10, and the channel walls 11 and 12 of the flow cell are formed of a transparent material. At the target adsorption position 14 installed in the channel walls 11 and 12 of the detection unit, the laser light emitted from under the channel 10 through the laser light source 22 and the condenser lens 21 is condensed. At this time, in order to be able to irradiate the target adsorption position 14 with laser light, the target adsorption position 14 is preferably made of a material that is substantially transparent to the wavelength of light emitted by the labeling substance of the magnetic particle conjugate captured to the adsorption portion in the flow cell and is preferably made of, for example, glass or plastic.

In addition, for example, in a case where the laser light source 22 and the condenser lens 21 are configured such that laser light need not be transmitted through the target adsorption position 14 by devising the arrangement and the like, it is preferable that the target adsorption position 14 is made of a material having excellent mechanical strength, corrosion resistance, processing efficiency, and the like, such as gold, platinum, or carbon, in consideration of the adsorption of the magnetic particle conjugate on the upper surface of the target adsorption position 14. If it is not necessary for any laser beam to be transmitted through the target adsorption position 14, the channel wall 12 need not be a transparent material, and a material such as ceramics, metal, or plastic can be used.

When light emission is to be measured, the fluid in the channel 10 is stopped in advance, and the magnet 15 is removed from the position immediately below the channel 10 by the slide mechanism 20 to cancel the magnetic force in the channel 10. This makes it possible to cause light emission by laser light irradiation while holding the magnetic particles 13 at the target adsorption position 14. Measurement can be performed by receiving the light emitted from the labeling substance bound to the magnetic particles 13 by the photodetector 23,

The controller 50 is connected to the valve 36, a valve 37, the pump 35, the arm 31, the laser light source 22, a photodetector 23, and the slide mechanism 20 by the signal lines 51, 52, 53, 54, 55, 56, and 57 so that they can be independently controlled.

As a labeling substance in this modification (FIG. 2), a labeling substance different from the labeling substance in the first embodiment (FIG. 1) may be used. Specific examples of labeling substances can be appropriately designed by those skilled in the art based on known techniques and the like.

In the immunoassay apparatus in FIG. 2, the controller 50, the arm 31, the slide mechanism 20, the pump 35, the valves 36 and 37, the suspension vessel 40, the cleaning liquid vessel 42, the buffer solution vessel 43, the channel 10, the magnetic particles 13, the labeling substance, and the like are as in the immunoassay apparatus in FIG. 1.

The operation of the sample analyzer according to the present embodiment will be described next using the immunoassay apparatus in FIG. 1 as an example.

One cycle of analysis is constituted by a suspension aspiration period, a magnetic particle capturing period, a detection period, a cleaning period, a reset period, and a preliminary aspiration period. One cycle is started when the suspension vessel 40 containing the suspension treated in the reaction unit 41 is set at a target position.

During a suspension aspiration period, it is set to a state wherein the valve 36 is opened and the valve 37 is closed. The arm 31 operates in accordance with signals from the controller 50 to insert the sipper nozzle 30 into the suspension vessel 40. Subsequently, the pump 35 performs a certain amount of aspiration operation in response to a signal from the controller 50. Aspirated by the fluid in the tube 32, the suspension in the suspension vessel 40 enters the tube 32 through the sipper nozzle 30. In this state, the pump 35 is stopped, and the arm 31 is operated to insert the sipper nozzle 30 into the cleaning mechanism 44. When passing through the cleaning mechanism 44, the sipper nozzle 30 is cleaned.

During a magnetic particle adsorption period, the slide mechanism 20 operates in response to a signal from the controller 50, and the magnet 15 moves to under the channel 10. The pump 35 aspirates at a constant speed in response to a signal from the controller 50. Meanwhile, the suspension present in the tube 32 passes through the channel 10. Since a magnetic field from the magnet 15 is generated in the channel walls 11 and 12, the magnetic particles 13 contained in the suspension are aspirated toward the magnet 15 by magnetic force and are trapped (captured) at the target adsorption position 14. After a lapse of a certain time, the aspiration of a suspension by the pump 3 is stopped.

In a detection period, the slide mechanism 20 is operated, and the magnet 15 is moved away from the channel 10. The controller 50 controls the voltage applying unit 18 to apply a voltage between the reaction field electrode 16 and the counter electrode 17 in the channel 10. This makes it possible to electrochemically emit light from the labeling substance bound to the magnetic particles 13 captured on the reaction field electrode 16 (the target adsorption position 14). The emission is optionally wavelength-selected by a filter and detected by the photodetector 23 such as a camera or a photomultiplier tube. The detected intensity of light emission is recovered by the controller 50 as a signal. After a lapse of a certain time, the voltage application is stopped. During a detection period, the arm 31 is operated to insert the sipper nozzle 30 into the cleaning mechanism 44.

During a cleaning period, a cleaning liquid aspirated from the cleaning liquid vessel 42 is caused to pass through the channel 10 by aspiration using the pump 35. At this time, since the magnet 15 is away from the channel 10, the magnetic particles 13 are not held at the target adsorption position 14 and are caused to flow away together with the cleaning liquid.

In a reset period, the valve 36 is closed, the valve 37 is opened, and the pump 35 is operated to discharge a liquid. The liquid in the pump 35 is discharged to the waste liquid vessel 45.

In a preliminary aspiration period, a buffer solution is aspirated from the buffer solution vessel 43, and the tube 32 and the channel 10 are filled with the buffer solution. After the preliminary aspiration period, the next cycle becomes executable.

As described above, the sample analyzer according to the present embodiment includes

- the channel configured to introduce the sample liquid containing the magnetic particles 13 bound to the specific substance (the substance to be analyzed) into the capturing region (the target adsorption position 14),
- the supply unit (the pump 35) configured to supply the sample liquid to the channel,
- the capturing unit (the magnet 15) having a magnetic field structure for generating a magnetic field and configured to adsorb the magnetic particles 13 to the capturing region by the magnetic field,
- the measurement unit (the photodetector 23) configured to measure the specific substance captured to the capturing region, and
- the discharge unit (the pump 35) configured to discharge the magnetic particles from the channel after measurement by the measurement unit.

In the present embodiment, the pump 35 is used for both the supply unit and the discharge unit, but individual constituent elements may be used for them. In this case, the sample liquid is a liquid containing a substance to be analyzed and is, for example, a suspension.

In addition, instead of the configurations described above with reference to FIGS. 1 and 2, a part of a known sample analyzer may be used.

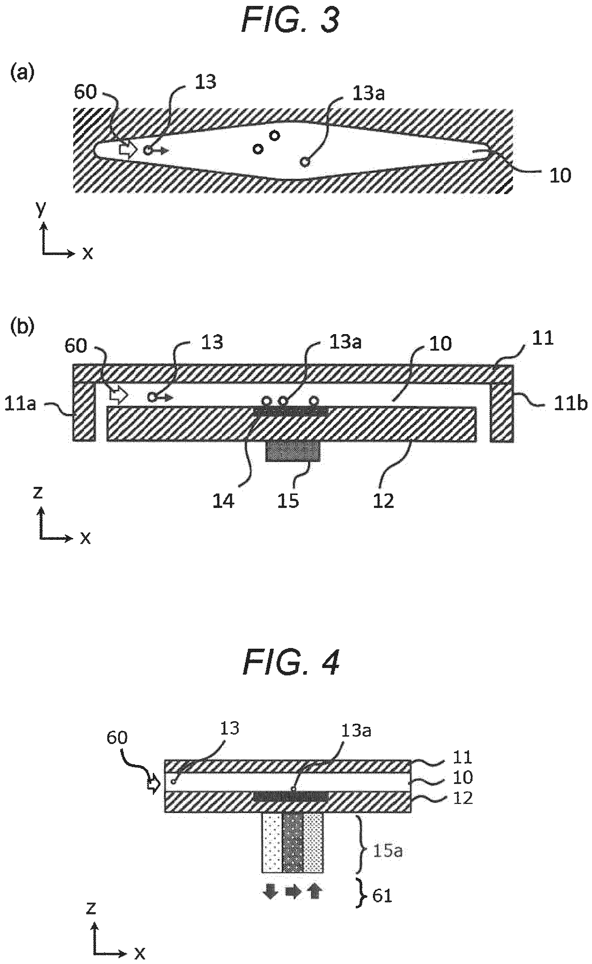

With regard to the sample analyzer described above, the structure around the target adsorption position 14 and the magnet 15 will be described with reference to FIGS. 3, 4, 5, and 6. The structure described below is applicable to both the first embodiment (FIG. 1) and the modification (FIG. 2) described above.

FIG. 3 is an enlarged view of a configuration near the channel of the immunoassay apparatus, showing a cross-section of an xy plane (FIG. 3(a)) and a cross-section of a zx plane (FIG. 3(b)) when a coordinate system is set, in which the traveling direction of a flow in a central portion of the flow cell is the x-axis, the horizontal direction of a cross-section perpendicular to the flow is the y-axis, and the vertical direction is the z-axis.

The suspension containing the magnetic particles 13 travels in the +z direction along the path formed by the channel walls 11a and 12 in FIG. 3 (b) and enters the flow cell. The suspension then travels in the + x direction as per a flow direction 60 in the channel. Upon reaching an end of the channel 10, the liquid travels in the -z direction along the path formed by the channel walls 11b and 12 and is discharged from the flow cell. During this time, the suspension receives the magnetic field of the magnet 15, and the magnetic particles 13 are trapped (captured) on the surface of the target adsorption position 14 by the magnetic force (captured magnetic particles 13a).

FIG. 4 is an enlarged view near the channel when the magnet structure according to the present embodiment is used. In the present embodiment, the magnet 15 includes a plurality of magnets 15a arranged outside the channel and having different magnetization directions. A magnetic field structure is formed by the plurality of magnets 15a. That is, the magnetic field structure includes the plurality of magnets 15a arranged outside the channel and respectively having different magnetization directions.

FIG. 4 shows a configuration in which permanent magnets are used as the magnets 15a, three magnets 15a having the same size are arranged in a line along the x direction without any gap, and the magnetization directions of the magnets 15a are the -z, +x, and +z directions, respectively, from the individual arranged upstream of the channel 10. In the channel 10, the suspension flows from upstream to downstream. That is, when one magnetic particle 13 flowing with the suspension is at the first position at the first point of time and at the second position at the second point of time after the first point of time, the first position is upstream with respect to the second position, and the second position is downstream with respect to the first position. In this arrangement, sandwiching the magnet in the middle, the magnets on the upstream side and the downstream side of the channel 10 generate magnetic fluxes in opposite directions.

In this case, a magnetization direction 61 of each magnet is indicated by an arrow so as to be S pole → N pole inside the magnet material (N pole → S pole outside the magnet such as air) according to a general magnetic flux notation.

A general magnet material can be used for the magnet 15a, and a material such as a ferrite magnet, a neodymium magnet, a samarium-cobalt magnet, an alnico magnet, or a combination thereof may be used.

The plurality of magnets 15a juxtaposed in the above-described form may be bonded to each other with an adhesive such as a curable resin or may be fixed by a jig or the like made of a non-magnetic material such as plastic or ceramics.

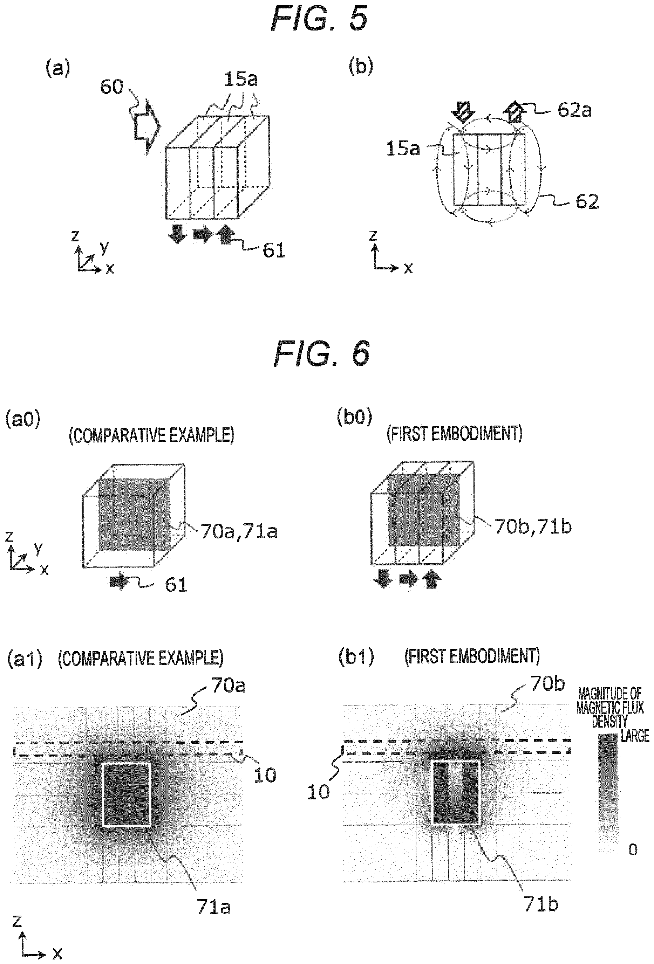

FIG. 5 shows a magnet structure and generated magnetic fluxes according to the present embodiment. One of the effects of the present embodiment will be described with reference to FIG. 5. FIG. 5(a) shows a magnet structure three-dimensionally showing the form in FIG. 4. In this form, FIG. 5(b) is a diagram showing a magnetic flux line 62 generated along the magnetization direction 61 in a magnet cross-section of the zx plane. The magnetic flux lines 62 have a loop shape, and for example, in the three magnets 15a, arrow directions of -z, +x, and +z are indicated from the individual arranged on the upstream side of the channel 10, similarly to the magnetization directions 61.

In the present embodiment, the three magnets 15a each form the same rectangular parallelepiped shape and are arranged such that each surface is orthogonal to the x-axis, the y-axis, or the z-axis. Note that the specific configuration is not limited to that shown in FIG. 5, and for example, a known Halbach array may be used.

By arranging the magnets 15a having the different magnetization directions 61, the magnetic flux lines 62 of the different magnets overlap each other so as to strengthen each other in partial regions outside the magnets. In particular, on the vertically upper side of the magnet, the directions of the magnetic flux lines 62 formed by the central magnet among the three magnets 15a and the other two magnets at the end potions are matched, so that the magnetic flux vectors are combined and the generated magnetic fluxes are intensified. According to the present embodiment, the magnetic fluxes in the directions of -z and +z are strengthened on the vertically upper side of the magnet as indicated by directions 62a of the intensified magnetic fluxes.

As described above, the plurality of magnets 15a are disposed such that magnetic fluxes generated by two adjacent magnets intensify each other at at least a part of the target adsorption position 14. The expression "magnetic fluxes intensify each other" means that, for example, vectors of magnetic fluxes intensify each other. Alternatively, this means that the signs of the z-direction components of the magnetic flux vectors coincide with each other.

As described above, in the arrangement in FIGS. 4 and 5, the plurality of magnets 15a are arranged such that the magnitude of the magnetic flux density on the channel side (for example, the magnetic flux density on the magnet surface on the channel side) is larger than the magnitude of the magnetic flux density on the side opposite to the channel (for example, the magnetic flux density on the magnet surface on the side opposite to the channel).

As a result, as an effect of the present embodiment, the magnetic field applied to the channel 10 can be strengthened, and the capturing rate of the magnetic particles 13 can be increased. In this case, the capturing rate is the value obtained by taking the ratio of the number of magnetic particles 13a captured to the target adsorption position 14 to the number of magnetic particles 13 included in the sample.

According to NPL 1, the force applied by the magnetic field to the substance in the magnetic field is generally known by the following equation.

$$\mathrm{F} = -\mathrm{grad}(\mathrm{E}) = (1/\mu_0)\chi\mathrm{B}(\mathrm{dB}/\mathrm{dl})$$

where F is the force vector, E is the energy vector, grad () is the operator representing the spatial gradient, $\mu_0$ is the vacuum permeability, $\chi$ is the magnetic susceptibility, B is the magnetic flux density vector, and 1 is the position.

In the strengthened magnetic field, the vector B of the magnetic flux density increases, and the force acting on the magnetic particles 13 also increases. In particular, in the configuration in FIG. 5, since the magnetic flux densities of the -z and + z components are enhanced and function with respect to the force of the vertical component, there is an effect that the capturing rate of the magnetic particles 13 is improved when it is desired to trap the magnetic particles 13 on the bottom surface of the channel 10.

In order to confirm the increase in magnetic flux density, the magnitude |B| of the magnetic flux density was calculated by magnetic field analysis (FIG. 6) (hereinafter, the magnitude of the magnetic flux density is indicated by |B| unless otherwise specified.). The magnetic field analysis was performed by a general-purpose magnetic field analysis method based on Maxwell's equations and Ampere's law.

FIG. 6(a0) shows a magnet model of one individual magnetized in the +x direction prepared for comparison, with the outside of the magnet being filled with air. The analysis results are shown in FIG. 6(a1). In this case, a surface 70a subjected to the magnetic field analysis was defined as a magnet cross-section parallel to the zx plane, and the position of the surface 70a in the y direction was defined as the central position of the magnet. According to the analysis result, a distribution was obtained in which the magnetic flux density is large in the air region near the position 71a of the magnet, and the magnetic flux density isotropically decreases as the distance from the magnet increases.

FIG. 6(b0) shows a model according to the first embodiment of the present invention. The magnet had the same material and outer size as those in FIG. 6(a0), and had the arrangement in FIG. 5. Three magnets 15a having the same size are arranged in a line along the x direction without any gap. A surface 70b subjected to the magnetic field analysis was taken as a magnet cross-section of the zx plane.

The results of the magnetic field analysis are shown in FIG. 6 (b1) . From the analysis result, as the same tendency as that of the magnet model of one individual in FIG. 6 (a1), a distribution was observed in which the magnetic flux density was large in the air region near the position 71b of the magnets, and the magnetic flux density decreased as the distance from the magnet increased. Referring to FIG. 6(b1), the obtained result was that the magnetic flux density on the vertical upper surface of the magnet was larger than that on the vertical lower surface.

The rapid change in magnetic flux density also indicated that the gradient (dB/dl) of the magnetic flux density in FIG. 6(b1) was larger than that in FIG. 6(a1). Although the channel 10 shown in FIGS. 6 (a1) and 6 (b1) indicates a case where the channel 10 is installed at a position vertically above the magnet surface, it can be seen that the present embodiment in FIG. 6(b1) generates a larger magnetic flux density in the channel than the case in FIG. 6(a1) (single magnet). According to the present embodiment, it was confirmed that the magnetic flux density and its gradient were increased.

For example, when the magnet outer dimension is 1 mm to 10 mm, the effect of increasing the magnetic flux density and its gradient is very strongly observed at a position of 0 mm to 1 mm in the +z direction from the upper surface of the magnet and is well observed at a position of 1 mm to 20 mm in the +z direction from the surface of the magnet. However, the material and dimensions of the magnet or the position and shape of the channel are not limited, and a higher effect can be obtained by appropriately designing the position where the magnetic flux density is desired to be increased according to the configuration of the sample analyzer.

According to the present embodiment, it is possible to reduce the magnetic flux density on the side opposite to the channel 10 on the magnet surface and to increase the magnetic flux density on the channel 10 side. Focusing on the target channel 10 side will obtain an effect of bringing out performance exceeding the original performance of the magnet material (the magnet model of one individual in FIG. 6(a1)). In addition, reducing the magnetic flux density on the surface on a specific side as in the vertical lower surface of the magnet in FIG. 6(b1) can reduce the magnetic noise with respect to the sample analyzer or another apparatus arranged adjacent thereto.

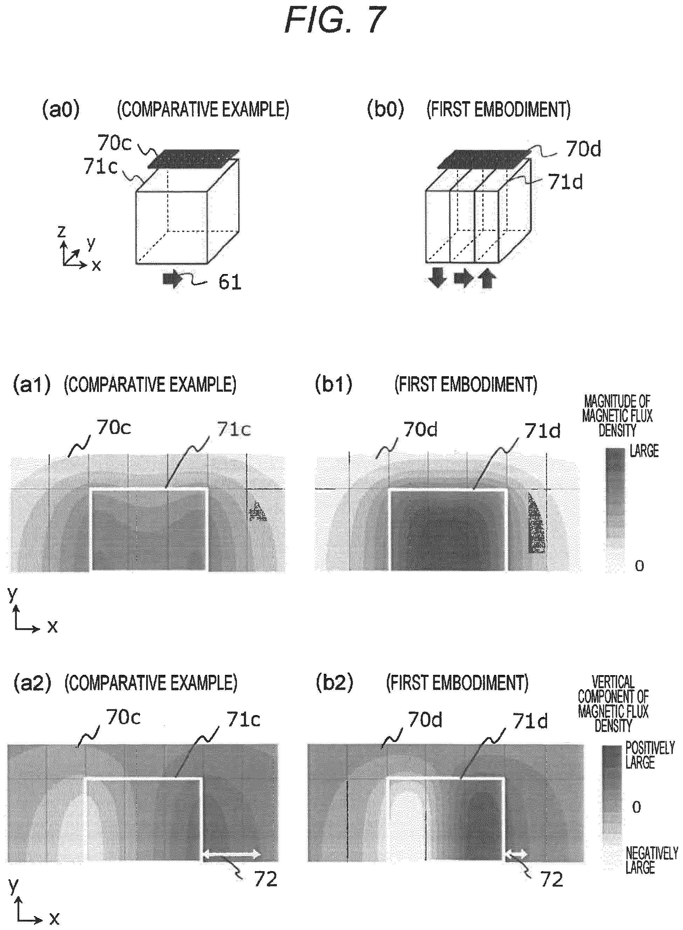

Next, focusing on the channel 10, the capturing rate of magnetic particles in the present embodiment and other accompanying effects will be described with reference to FIGS. 7, 8, 9, and 10.

FIG. 7 shows a magnet model and an analysis result when a magnetic field analysis is performed using the model and conditions similar to those in FIG. 6, focusing on the channel 10. FIG. 7(a0) shows a magnet model of one individual magnetized in the +x direction, and FIG. 7(b0) shows a magnet model having the three magnets 15a according to the present embodiment arranged without any gap.

Assuming that the channel 10 is vertically above each magnet, surfaces 70c and 70d subjected to the magnetic field analysis are defined in the air region vertically above the upper surfaces 71c and 71d of the magnets. The surfaces 70c and 70d are parallel to the xy plane.

With this arrangement, in the case in FIG. 7(b0), the magnetic particles 13 pass through the upper surfaces of the magnets of the plurality of individuals by flowing through the channel 10. In the present embodiment using the three magnets 15a, with regard to the magnet 15a (first magnet) on the most upstream side, the next magnet 15a (second magnet), and the further next magnet 15a (third magnet) (that is, the most downstream side), the magnets 15a are arranged such that the sample liquid passes through all of a region closer to the first magnet than the second magnet and the third magnet, a region closer to the second magnet than the first magnet and the third magnet, and a region closer to the third magnet than the first magnet and the second magnet. As described above, the three magnets 15a are arranged in parallel on the channel 10 in the direction in which the sample liquid flows, and the sample liquid passes above all the magnets 15a. A similar arrangement is possible in a case where two or four or more magnets 15a are used.

FIGS. 7(a1) and 7(b1) show the analysis results of the magnetic flux densities of the respective models. When the magnitudes of the magnetic flux densities are compared, FIG. 7 (b1) according to the present embodiment shows a larger value. In addition, it was also confirmed that the change in magnetic flux density near the magnet was also rapid, and the gradient was large. Therefore, by using the present embodiment, the magnetic flux density and the gradient thereof can be increased.

This tendency was also observed at the vertical components of the magnetic flux densities shown in FIGS. 7(a2) and 7(b2), and it was confirmed that the force in the vertical direction acting on the magnetic particles was increased by using the present embodiment. In a case where the target adsorption position 14 is provided on the bottom surface of the channel 10, the magnetic particles 13 in the channel 10 are captured to the target adsorption position 14 by the vertically downward magnetic force, so that the adsorption of the magnetic particles 13 can be promoted.

In this case, referring to FIGS. 7(a2) and 7(b2), focusing on the changes in magnetic flux density near the magnets, with regard to regions having relatively large vertical components of the magnetic flux densities (that is, regions whiter or darker than surrounding regions), when, for example, widths 72 of the same blackness ranges are compared, the width in FIG. 7(b2) according to the present embodiment is narrower. In the present embodiment, the magnetic flux density rapidly decreases as the distance increases from the region where the magnetic flux density on the upper surface of the magnet is large, and the region where the magnetic force acting on the magnetic particles is strong and the region where the magnetic force acting on the magnetic particles is weak are clearly divided. As a result, the magnetic particles 13 can be captured at a high density at the target adsorption position 14, and at the same time, adsorption to a place other than the target adsorption position 14 can be prevented. The controllability of an adsorption distribution can be enhanced, and uneven adsorption can be prevented.

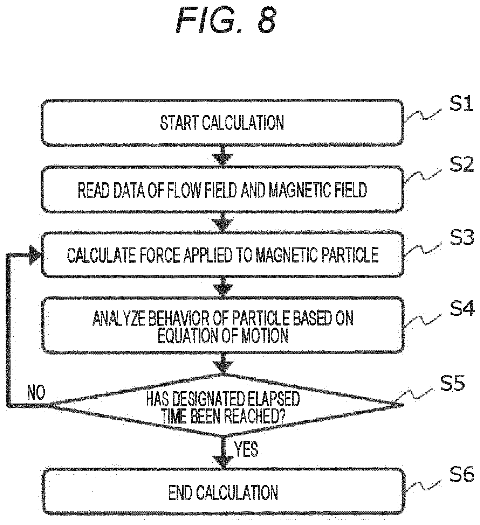

In order to confirm the effect of the present embodiment, the adsorption process was analyzed using a numerical simulator capable of analyzing the behavior of magnetic particles with high accuracy. FIG. 8 shows a flowchart of magnetic particle behavior analysis in a flow field and a magnetic field.

When the calculation is started (S1), the fluid in the channel 10 is analyzed using general-purpose fluid analysis software to obtain a velocity field and a pressure field. At the same time, a magnetic field around the magnet is separately obtained using general-purpose magnetic field analysis software. Next, the particle behavior analysis program reads the data of the flow field and the magnetic field (S2), and the force applied to each particle can be calculated using the values of the flow field and the magnetic field at each particle position (S3). In this case, as the force applied to each particle, the force received from the flow, the gravity force, and the force received from the magnet are evaluated. For each particle, Newton's equation of motion was sequentially solved to analyze the behavior of the magnetic particle by performing particle behavior analysis while updating the position of the magnetic particle (S4). The calculation is repeated until the designated elapsed time is reached (S5) . When the designated elapsed time is reached, the calculation is ended (S6).

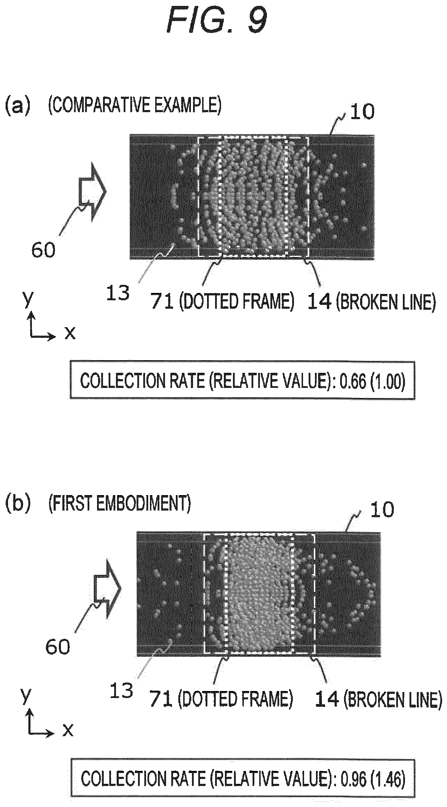

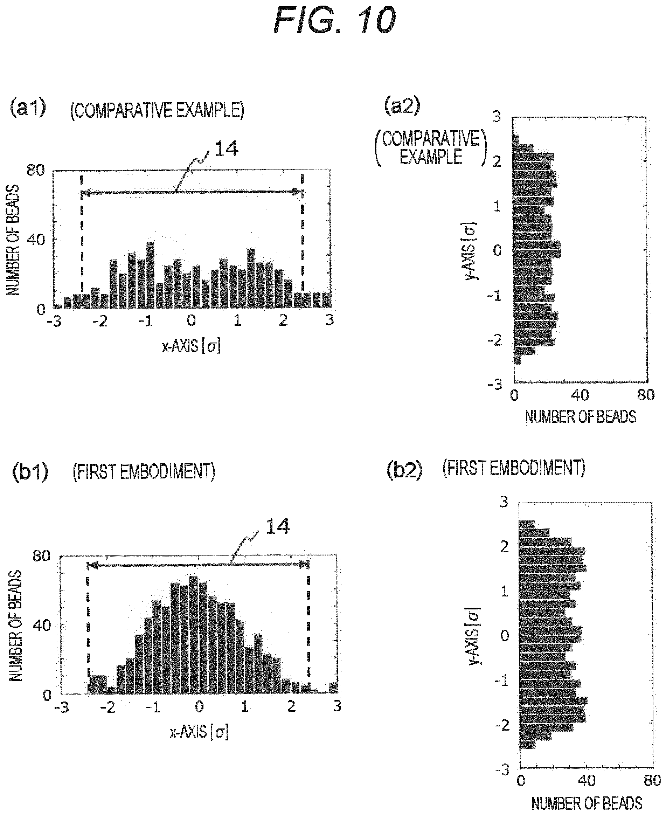

FIGS. 9(a) and 9(b) show the results of analyzing the adsorption distributions of magnetic particles in the case of using the magnets in FIGS. 7(a0) and 7(b0) in the channels 10. FIG. 10 is a histogram of the position distribution of captured magnetic particles along the x-axis and the y-axis based on a normalized length unit $\sigma$. FIGS. 9(a), 10(a1), and 10(a2) each show the adsorption distribution of magnetic particles in a magnet model of one individual, and FIGS. 9(b), 10(b1), and 10(b2) each show the adsorption distribution of magnetic particles in this embodiment.

When the numbers of captured magnetic particles in FIGS. 9(a) and 9 (b), FIGS. 10 (a1) and 10 (b1), and FIGS. 10 (a2) and 10 (b2) were compared with each other, the present embodiment obtained the result indicating that the capturing rate of magnetic particles was larger in the present embodiment. In this case, FIG. 9 shows the rate at which the magnetic particles 13 in the fluid are captured to the target adsorption position 14 as a capturing rate. The present embodiment in FIG. 9(b) exhibited a higher numerical value than the magnet of one individual in FIG. 9(a).

Referring to FIG. 9, when the adsorption positions of magnetic particles are compared for positions 71 of the magnets indicated by the dotted line, in the case of the magnet model of one individual (FIG. 9(a)), the magnetic particles are captured in the x-axis region wider than the position 71 of the magnet, and the increase and decrease in the number of magnetic particles gradually change depending on the position. On the other hand, in the case of the present embodiment (FIG. 9(b)), the position 71 of the magnets and the position where many magnetic particles are captured are almost the same region.

These can be confirmed from the histograms on the x-axis shown in FIGS. 10(a1) and 10(b1). In the case of the magnet model of one individual (FIG. 10(a1)), a certain number of adsorptions was also observed at a position out of the target adsorption position 14. However, in the case of the present embodiment (FIG. 10(b1)), the number of adsorptions at the position out of from the target adsorption position 14 was small. As described above, the controllability of the aspiration position is enhanced by the present embodiment.

Furthermore, for example, the measurement accuracy can be further improved by appropriately designing the shape and dimension of the target adsorption position 14 such as matching the dimension in the x direction of the target adsorption position 14 with the position 71 of the magnet.

According to the present embodiment described above, it is possible to provide a sample analyzer that achieves an increase in the capturing rate of magnetic particles, suppression of uneven adsorption by improving the controllability of the adsorption distribution of magnetic particles, or both.

Second Embodiment

As another embodiment of the present invention, an embodiment using a magnet different from that of the first embodiment will be described. In the second and subsequent embodiments, the sample analyzers each are the same as the sample analyzer according to the first embodiment except for a magnet such as a flow cell, and a detailed description thereof is omitted. The form described in each embodiment is not limited to any specific shape, dimensions, number, and the like, and can be changed without departing from the gist thereof, and a plurality of embodiments may be combined including the embodiments described above or later.

The plurality of magnets used in the present embodiment may be a combination of magnets having magnetization directions different from those in FIG. 5 as long as the resultant form can obtain an effect of increasing the capturing rate of magnetic particles or an effect of simultaneously increasing the capturing rate of magnetic particles and suppressing uneven adsorption by improving controllability of the adsorption distribution of magnetic particles.

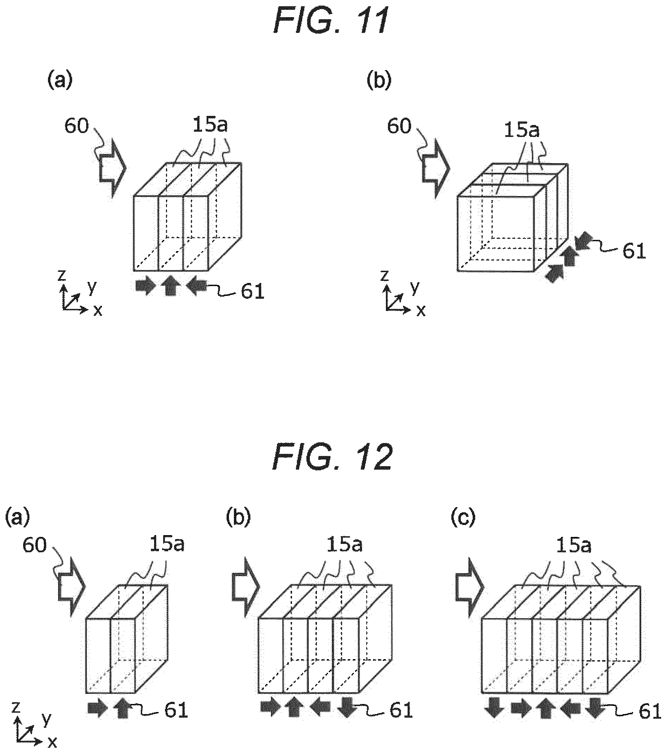

FIG. 11(a) shows an example in which a structure having three magnets 15a with the same size arranged in a line without any gap is configured by a combination of magnets having different magnetization directions. As shown in FIG. 11(a), even when the magnets are respectively arranged in the +x, +z, and -x magnetization directions from the individual on the upstream side of the channel, the magnetic flux vectors are strengthened each other to increase the magnetic flux density on the upper surface of each magnet. This will obtain an effect of increasing the capturing rate of the magnetic particles 13.

In the present embodiment, the magnetization directions of the adjacent magnets are basically different from each other by 90°, but the magnetic flux vectors can be strengthened by an arbitrary relative difference except that the magnetization directions of the adjacent magnets are the same (the relative difference in angle is 0°). When the relative difference in angle is defined in the range of -90° to +90°, the absolute value of the relative difference in angle may be any value larger than 0°, and may be preferably 45° or more, more preferably 75° or more, and still more preferably 85° or more. When the absolute value of the relative difference is set to 90°, it is easy to obtain the effect that the magnetic flux density is larger on one surface (the upper surface of the magnet) of the magnet than on its opposite surface (the lower surface of the magnet). However, the absolute value of the relative difference may be set to less than 90° depending on the shape of the target adsorption position 14 and the target capturing rate and trap distribution.

In addition, in a case where the number of magnets is three or more, the relative differences in angle in the magnetization directions between all the adjacent magnets need not be the same, and angles having different relative differences may be used in combination at a plurality of adjacent positions. This makes it possible to control the degree of mutual strengthening of the magnetic fluxes generated in the channel 10 and the degree of mutual weakening of the magnetic fluxes in the space on the opposite side to the channel 10.

In particular, in the embodiment in which three or more magnets are arranged in a line, as exemplarily shown in FIGS. 5(a) and 11(a), a portion and a region where magnetic fluxes strengthen each other can be increased by a structure in which another magnet is arranged between two magnets from which opposite magnetic fluxes are generated.

Note that the modification of the relative difference in the magnetization direction can be applied not only to the present embodiment but also to other embodiments.

The plurality of magnets may be arranged adjacent to each other across the channel 10. For example, as shown in FIG. 11(b), the three magnets 15a having the same size may be arranged in a line along the y direction without any gap. The plurality of magnets 15a are arranged in parallel in a direction perpendicular to the direction in which the sample liquid flows in the channel 10, and the sample liquid passes above the different magnets.

That is, in this case, it can be said that the channel (for example, the target adsorption position 14 and the space above the target adsorption position) has the right side region, the middle region, and the left side region in the direction crossing the channel, and the plurality of magnets 15a can be said to be arranged such that the sample liquid passes above the different magnets 15a in the right side region, the middle region, and the left side region.

This embodiment also obtains an effect of increasing the magnetic flux density on the upper surface of each magnet and an effect of increasing the capturing rate of the magnetic particles 13. In particular, the generated magnetic field distribution in the y direction can be controlled by the configuration in FIG. 11(b). Therefore, this configuration is effective when the distribution of the captured magnetic particles 13a is optimized in the y direction according to the arrangement form of the mechanism that causes the fluorescent labeling substance to emit light after the magnetic particles 13 are trapped. For example, in a mechanism using a reaction field electrode 16 and a counter electrode 17, when the counter electrode 17 is disposed along the y direction, it is possible to perform control so that the magnetic particles 13 are sufficiently distributed and captured in the central portion of the channel or near a side surface (vertical wall surface parallel to the channel in the flow cell) of the channel.

The adjacent arrangement of the plurality of magnets is based on a direction parallel or perpendicular to the channel 10, but the magnets may be arranged along other directions depending on the shape or arrangement of the target adsorption position 14 or the mechanism. For example, in the arrangement and shape of the target adsorption position 14 shown in FIG. 9, when an arrangement in which the target adsorption position 14 is rotated by 45° in the xy plane about the center point of the channel is used, the arrangement of the plurality of magnets 15a may be similarly rotated.

The number of magnets may be other than three, and for example, FIG. 12 shows examples of structures in which (a) two magnets 15a, (b) four magnets 15a, or (c) five magnets 15a are arranged in a line along the x direction without any gap. Some of the effects of the present embodiment can be obtained without limiting to the number of magnets, and six or more magnets may be used. By increasing the number of magnets, the distribution of the generated magnetic field can be precisely controlled. On the other hand, when the number of magnets is small, the effect of the present embodiment can be obtained by a simple manufacturing method by omitting adhesion and fixing of the magnets.

In particular, when three or more magnets are included as the plurality of magnets, it is possible to flexibly control the formation of a magnetic field. When three or more magnets are used, the one-line arrangement is not essential, and for example, a plurality of magnets may be two-dimensionally arranged in the xy plane. When a plurality of magnets is juxtaposed in each of the directions x and y, the magnetic field in the channel 10 can be precisely controlled. Similarly, a plurality of magnets may be three-dimensionally arranged. Assume that a plurality of magnets (for example, three magnets including the first to third magnets) are three-dimensionally arranged. In this case, the magnets are arranged so that the sample liquid passes through all of a region closer to the first magnet than the second magnet and the third magnet, a region closer to the second magnet than the first magnet and the third magnet, and a region closer to the third magnet than the first magnet and the second magnet in the channel 10, whereby the effect of increasing the magnetic flux density on the upper surface of each magnet can be easily obtained. However, the magnets need not necessarily be arranged to make the sample liquid pass through the three regions as long as the magnets are arranged to obtain the effect of increasing the magnetic flux density on the upper surface of each magnet.

When only two magnets are used, the magnetization directions of the two magnets are not limited to the pattern shown in FIG. 12(a), and a pattern obtained by extracting any two adjacent magnetization directions from the five magnetization directions in FIG. 12(c) may be used. Furthermore, also in the case of using three or more magnets, a pattern obtained by extracting any two adjacent magnetization directions from the five magnetization directions in FIG. 12(c) may be used.

As described above, it is possible to provide a sample analyzer that achieves an increase in the capturing rate of magnetic particles, suppression of uneven adsorption by improving the controllability of the adsorption distribution of magnetic particles, and both by using the magnet arrangement different from that in the first embodiment.

Third Embodiment

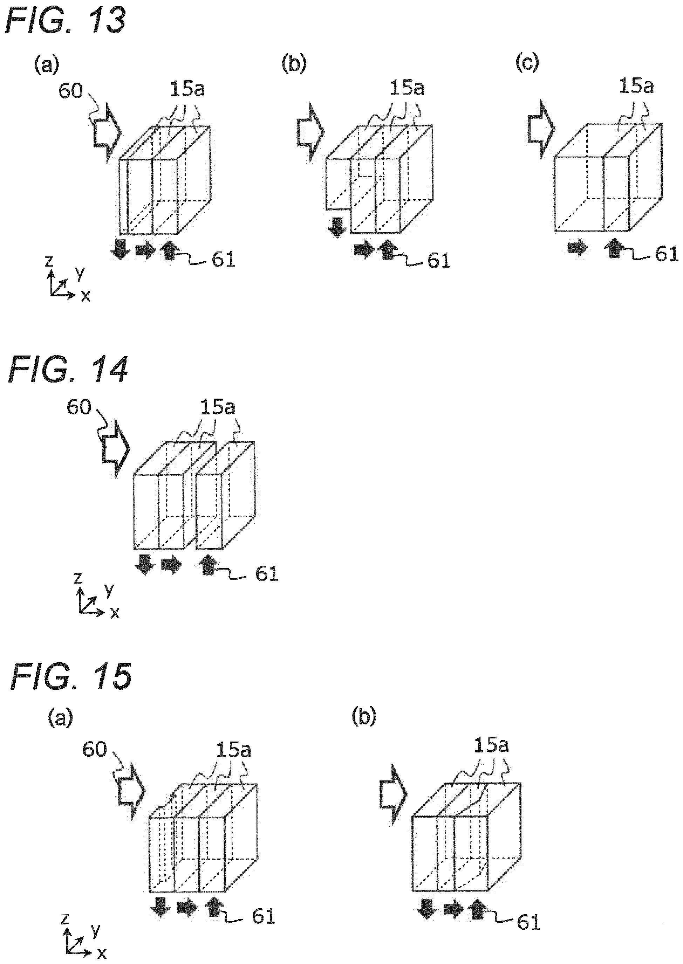

The plurality of magnets used in the present embodiment may be magnets having different dimensions, and for example, two or more magnets having different dimensions on the x-, y-, or z-axis may be arranged in a line along the x direction without any gap. FIG. 13 shows an example of a structure in which the magnet arranged on the upstream side of the channel is smaller than the magnet arranged on the downstream side of the channel (FIGS. 13(a) and 13(b)) and an example of a structure in which the magnet that generates the magnetic field in the vertical direction is arranged only on the downstream side of the channel (FIG. 13(c)).

In the sample analyzer exemplified by the first embodiment, in the process of capturing the magnetic particles 13 from the fluid, the magnetic particles first approach the upstream side of the channel of the magnet, and the number of particles captured on the upstream side of the channel, that is, the density of the captured magnetic particles, increases. As a result, for example, in a case where a magnet of one individual is used (FIGS.

6(a) and 7(a)), the number of particles captured on the downstream side of the channel becomes relatively small, so that the evenness of the magnetic particle distribution at the target adsorption position 14 decreases.

FIGS. 13(a) and 13(b) show a structure in which the magnet arranged on the channel upstream side is smaller than the magnet arranged on the channel downstream side, and the magnetic field generated on the channel downstream side is larger than the magnetic field generated on the channel upstream side. As a result, it is possible to suppress a decrease in evenness between the upper and lower portions of the channel, which is likely to occur when a single magnet is used, and to improve the evenness of the magnetic particle distribution at the target adsorption position 14.

The magnet on the upstream side of the channel at this time may be reduced in dimension in a direction (the x direction in FIG. 13(a)) parallel to a flow direction 60 in the channel as shown in FIG. 13 (a), may be reduced in dimension in a direction (the y direction or the z direction) perpendicular to the flow direction 60 in the channel as shown in FIG. 13(b), or may be a combination thereof.

By arranging the vertically upper surface of the magnet on the upstream side of the channel and the vertically upper surface of another magnet on the same plane, the magnetic field generated on the downstream side of the channel can be made larger than the magnetic field generated on the upstream side of the channel without weakening the magnetic field generated on the upstream side of the channel (without impairing the superiority of the magnet material and arrangement). This makes it possible to implement measurement in an environment in which adsorption of magnetic particles is more difficult, such as a high-speed fluid and a channel 10 having a large area cross-section.

However, the vertically upper surface of the magnet on the upstream side of the channel and the vertically upper surface of the other magnets need not necessarily be disposed on the same plane. For example, in order to improve the evenness of the magnetic particle distribution, when it is desired to make the magnetic field generated on the downstream side of the channel larger than the upstream side of the channel, it can be realized by separating the vertically upper surface of the magnet on the upstream side of the channel to a position farther away from the channel than the vertically upper surface of another magnet.

FIG. 13(c) shows a structure using a plurality of (two) magnets 15a having different areas of the surfaces facing the channel. In particular, a magnet having a large dimension in the x direction is used as the magnet on the upstream side of the channel, and a magnet that generates a magnetic field in the vertical direction is disposed only on the downstream side of the channel. The plurality of magnets 15a include a first magnet and a second magnet that differs in the area of a surface facing the channel from that of the first magnet. In this structure, since the magnetic fluxes intensify each other only on the downstream side of the channel, the magnetic flux density on the downstream side can be made larger than that on the upstream side of the channel. Since the size of the magnet on the upstream side of the channel is large, it is possible to caupure the magnetic particles on the upstream side with the same distribution and capturing rate as in the case of using one individual magnet. On the other hand, on the downstream side of the channel, magnetic particles that cannot be collected and flow out when one individual magnet is used are captured inside the target adsorption position 14 by a strong magnetic field, and the capturing rate can be improved and the evenness can be improved.

In the third embodiment described above, the regions referred to as "upstream" and "downstream" may be defined with respect to the space of the flow channel or may be defined with respect to the bottom surface of the flow channel (same for other embodiments). In the case of being defined with respect to the space, it can be said that the plurality of magnets 15a are arranged such that the magnetic flux density on the downstream side is larger than the magnetic flux density on the upstream side in the space in the channel above the target adsorption position 14. On the other hand, in the case of being defined with respect to the bottom surface, it can be said that the plurality of magnets 15a are arranged such that the magnetic flux density on the downstream side is larger than the magnetic flux density on the upstream side on the bottom surface of the channel (for example, the portion constituting the target adsorption position 14).

Fourth Embodiment

With regard to the plurality of magnets used in the present embodiment, there may be a gap between the magnets. FIG. 14 shows a structure in which three magnets 15a are arranged in a line along the x direction with a gap between some magnets. At this time, the gap may be formed of air, a resin material, ceramics, metal, or the like. The gap can be used, for example, for fixing arbitrary magnets with an adhesive or a jig made of ceramics or metal.

The gap between two adjacent magnets can be sized such that magnetic fields are mutually strengthened by combining magnetic flux vectors. Assuming that two adjacent magnets are arranged along the x-axis, in order to increase the magnetic flux density and gradient, the minimum distance (interval) between the magnets is preferably smaller than the larger x-axis dimension (denoted as Lx) of both magnets. That is, the plurality of magnets 15a are preferably arranged such that the interval between two adjacent magnets is smaller than the width (the dimension in the array direction) of any of the two adjacent magnets.

From the same viewpoint, the minimum distance (interval) between the magnets is more preferably smaller than Lx/2. In particular, like the two magnets on the upstream side shown in FIG. 14, it is further preferable to bring the magnets into contact with each other in order to increase the magnetic flux density and the gradient.

Fifth Embodiment

The shape of each of plurality of magnets used in the present embodiment is not limited to a rectangular parallelepiped and may be, for example, a shape including a unique portion such as a groove or an irregular portion. FIG. 15 shows a structure in which at least one magnet 15a having a shape similar to a groove or an irregular portion is used, and three magnets 15a are arranged in a line along the x direction.

FIG. 15 (a) shows a form in which, in the structure (FIG. 5(a)) in which the three magnets 15a having the same size are arranged in a line without any gap, a groove is formed in the middle portion in the y direction of the magnet positioned on the upstream side of the channel. By making the magnet on the upstream side of the channel smaller by a volume corresponding to the groove, the magnetic field generated on the downstream side of the channel can be made larger than the magnetic field generated on the upstream side of the channel, and the evenness of the magnetic particle distribution can be improved. In addition, by forming a groove extending in the z direction in the middle portion in the y direction, it is possible to strengthen the magnetic fields on the side wall sides (both sides in the y direction) with respect to the middle portion of the channel. With such a structure, the controllability of the magnetic particle distribution in the y direction can be improved.

In the example in FIG. 15(a), it can be said that the channel (for example, the target adsorption position 14 and the space above the target adsorption position) has a right side region, a middle region, and a left side region in a direction crossing the channel (particularly, a direction crossing horizontally), and at least one (upstream magnet) of the plurality of magnets has a shape in which the magnetic flux densities generated in the right side region and the left side region by the magnet are larger than the magnetic flux density generated in the middle region by the magnet.

By forming a groove or an irregular portion in a sharp shape (for example, including a 90° irregular portion), the magnetic flux density gradient from the magnet to the air region can be increased, which is useful for designing a flow cell in which the capturing rate is desired to be improved. In particular, from the viewpoint of improving the capturing rate, for example, it is desirable to use the configuration of the present embodiment near the interfaces between the target adsorption position 14 and other regions.

FIG. 15 (b) shows a form in which, in the structure (FIG. 5(a)) in which the three magnets 15a having the same size are arranged in a line without any gap, irregular portions which fit each other are formed on the magnets respectively positioned in the middle and the downstream of the channel. Two adjacent magnets face each other with surfaces shaped to fit each other. Note that, in the example in FIG. 15(b), the magnets face each other and are in contact with each other, but may be arranged to face each other and be separated from each other as shown in FIG. 14.

The middle magnet has a shape in which both sides in the y direction of the +x side surface of the rectangular parallelepiped are removed to form a convex portion in the +x direction and has a volume smaller than that of the original rectangular parallelepiped. The magnet on the downstream side has a shape with a concave portion facing the -x direction, which is formed by making both the y-direction sides of the -x side surface of the rectangular parallelepiped protrude toward the -x side, and has a larger volume than the original rectangular parallelepiped. As described above, by enlarging the magnet on the downstream side of the channel, the magnetic field generated on the downstream side of the channel can be made larger than the magnetic field generated on the upstream side of the channel, and the evenness of the magnetic particle distribution can be improved as compared with the x-axis method.

By forming a groove or an irregular portion in a gentle shape (for example, including an irregular portion with 45° or less), it is possible to suppress a rapid change in the magnetic field in a region where the magnets are adjacent as compared with a case where the groove or the irregular portion is formed in a sharp shape, which is useful for designing a flow cell in which evenness is desired to be improved. This is particularly related to the distribution at the site where the magnetic particles are captured, and thus it is desirable to use this embodiment inside the target adsorption position 14.

In the above-described embodiments and modifications, the plurality of magnets may include permanent magnets or electromagnets. When a permanent magnet is used, heat generation can be suppressed. When an electromagnet is used, a magnetic field is erased when the magnetic field is unnecessary, and the influence of the magnetic field on the peripheral configuration can be suppressed.

The plurality of magnets may include a permanent magnet and an electromagnet. Alternatively, the plurality of magnets may include a permanent magnet made of a first material and a permanent magnet made of a second material different from the first material. The material is, for example, neodymium, ferrite, samarium cobalt, or alnico, and one of them can be appropriately selected based on a known technique or the like. By using magnets of different configurations in this manner, it is possible to more flexibly control the magnetic field at each position.

Although the embodiments and modifications of the present invention have been specifically described above, the present invention is not limited to the above-described embodiments and modifications and can be modified without departing from the gist thereof. According to the embodiments and modifications of the present invention, it is possible to provide a sample analyzer that achieves an increase in the capturing rate of magnetic particles, suppression of uneven adsorption by improving the controllability of the adsorption distribution of magnetic particles, and both.

Reference Signs List

10 channel
11, 12 channel wall
11a, 11b channel wall
13 magnetic particle
13a captured magnetic particle
14 target adsorption position (capturing region)
15, 15a magnet
16 reaction field electrode
17 counter electrode
18 voltage applying unit
19a, 19b lead wire
20 slide mechanism
21 condenser lens
22 laser light source
23 photodetector
30 sipper nozzle
31 arm
32, 33, 34 tube
35 pump
36, 37 valve
40 suspension vessel
41 reaction unit
42 cleaning liquid vessel
43 buffer solution vessel
44 cleaning mechanism
45 waste liquid vessel
50 controller
51, 52, 53, 54, 55, 56, 57, 58 signal line
60 flow direction in channel
61 magnetization direction
62 magnetic flux line
62a intensified magnetic flux direction
70a, 70b, 70c, 70d surface subjected to magnetic field analysis
71, 71a, 71b position of magnet
71c, 71d upper surface of magnet
72 width of region where vertical component of magnetic flux density is relatively large

All publications, patents, and patent applications cited herein are hereby incorporated by reference in their entirety.

**Claims**

**1.** A sample analyzer comprising:

a channel configured to introduce a sample liquid containing magnetic particles bound to a specific substance into a capturing region;
a supply unit configured to supply the sample liquid to the channel;
a capturing unit having a magnetic field structure for generating a magnetic field and configured to adsorb the magnetic particles to the capturing region by the magnetic field;
a measurement unit configured to measure the specific substance captured to the capturing region; and
a discharge unit configured to discharge the magnetic particles from the channel after measurement by the measurement unit,
wherein the magnetic field structure includes a plurality of magnets arranged outside the channel and having different magnetization directions, the plurality of magnets being arranged so as to make a magnetic flux density on a channel side larger than a magnetic flux density on a side opposite to the channel.

2. The sample analyzer according to claim 1, wherein the plurality of magnets are arranged so as to make magnetic fluxes generated by two adjacent magnets intensify each other in at least a part of the capturing region.

3. The sample analyzer according to claim 1, wherein the plurality of magnets are arranged in parallel in a direction in which the sample liquid flows in the channel, and the sample liquid passes above all the plurality of magnets.

4. The sample analyzer according to claim 1, wherein the plurality of magnets are arranged in parallel in a direction perpendicular to a direction in which the sample liquid flows in the channel, and the sample liquid passes above different magnets.

5. The sample analyzer according to claim 1, wherein the plurality of magnets are arranged so as to make a magnetic flux density on a downstream side of the channel larger than a magnetic flux density on an upstream side of the channel in a space in the channel above the capturing region.

6. The sample analyzer according to claim 1, wherein the plurality of magnets include three or more magnets.

7. The sample analyzer according to claim 1, wherein the plurality of magnets are arranged so as to make a magnetic flux density on a downstream side of the channel larger than a magnetic flux density on an upstream side of the channel on a bottom surface of the channel constituting the capturing region.

8. The sample analyzer according to claim 1, wherein the plurality of magnets are arranged so as to make an interval between two adjacent magnets smaller than a width of any of the two adjacent magnets.

9. The sample analyzer according to claim 1, wherein the plurality of magnets are arranged so as to make two adjacent magnets face each other with surfaces having shapes to be fitted to each other.

10. The sample analyzer according to claim 5, wherein

the channel has a right side region, a middle region, and a left side region in a direction crossing the channel, and
at least one of the plurality of magnets has a shape in which a magnetic flux density generated in the right side region and the left side region by the magnet is larger than a magnetic flux density generated in the middle region by the magnet.

11. The sample analyzer according to claim 1, wherein the plurality of magnets include a permanent magnet.

12. The sample analyzer according to claim 1, wherein the plurality of magnets include an electromagnet.

13. The sample analyzer according to claim 1, wherein the plurality of magnets

- include a permanent magnet and an electromagnet, or
- include a permanent magnet made of a first material and a permanent magnet made of a second material.

14. The sample analyzer according to claim 1, wherein the plurality of magnets include a first magnet and a second magnet that differs from the first magnet in an area of a surface facing the channel.

# FIG. 1

FIG. 2

# FIG. 3

(a)

(b)

# FIG. 4

# FIG. 5

(a)

(b)

# FIG. 6

(a0) (COMPARATIVE EXAMPLE)

70a,71a

(b0) (FIRST EMBODIMENT)

70b,71b

(a1) (COMPARATIVE EXAMPLE)

70a

10

71a

(b1) (FIRST EMBODIMENT)

70b

10

71b

MAGNITUDE OF MAGNETIC FLUX DENSITY

LARGE

0

# FIG. 7

(a0)  (COMPARATIVE EXAMPLE)

70c
71c
z
y
x
61

(b0)  (FIRST EMBODIMENT)

70d
71d

(a1)  (COMPARATIVE EXAMPLE)

70c    71c

y
x

(b1)  (FIRST EMBODIMENT)

70d    71d

MAGNITUDE OF MAGNETIC FLUX DENSITY

LARGE

0

(a2)  (COMPARATIVE EXAMPLE)

70c    71c

72

y
x

(b2)  (FIRST EMBODIMENT)

70d    71d

72

VERTICAL COMPONENT OF MAGNETIC FLUX DENSITY

POSITIVELY LARGE

0

NEGATIVELY LARGE

# FIG. 8

START CALCULATION — S1

READ DATA OF FLOW FIELD AND MAGNETIC FIELD — S2

CALCULATE FORCE APPLIED TO MAGNETIC PARTICLE — S3

ANALYZE BEHAVIOR OF PARTICLE BASED ON EQUATION OF MOTION — S4

HAS DESIGNATED ELAPSED TIME BEEN REACHED? — S5
NO
YES

END CALCULATION — S6

# FIG. 9

(a) (COMPARATIVE EXAMPLE)

60

13　71 (DOTTED FRAME)　14 (BROKEN LINE)

Y
X

COLLECTION RATE (RELATIVE VALUE): 0.66 (1.00)

(b) (FIRST EMBODIMENT)

60

13　71 (DOTTED FRAME)　14 (BROKEN LINE)

Y
X

COLLECTION RATE (RELATIVE VALUE): 0.96 (1.46)

# FIG. 10

(a1)  (COMPARATIVE EXAMPLE)

(a2) (COMPARATIVE EXAMPLE)

(b1)  (FIRST EMBODIMENT)

(b2)  (FIRST EMBODIMENT)

# FIG. 11

(a)

(b)

# FIG. 12

(a)

(b)

(c)

## FIG. 13

(a)

60

15a

61

z
y
x

(b)

15a

61

(c)

15a

61

## FIG. 14

60

15a

61

z
y
x

## FIG. 15

(a)

60

15a

61

z
y
x

(b)

15a

61

# INTERNATIONAL SEARCH REPORT

| International application No. |
|---|
| **PCT/JP2022/027987** |

## A. CLASSIFICATION OF SUBJECT MATTER

*G01N 35/08*(2006.01)i
FI: G01N35/08 A

According to International Patent Classification (IPC) or to both national classification and IPC

## B. FIELDS SEARCHED

Minimum documentation searched (classification system followed by classification symbols)

G01N35/08, H01F7/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| X | JP 2013-238420 A (HITACHI HIGH-TECHNOLOGIES CORP) 28 November 2013 (2013-11-28)<br>claim 1, paragraphs [0016]-[0047], fig. 1 | 1-14 |
| A | JP 2004-535292 A (BIOMERIEUX SA) 25 November 2004 (2004-11-25)<br>claim 1, fig. 3 | 1-14 |
| A | WO 2020/061696 A1 (OCTANE BIOTECH INC.) 02 April 2020 (2020-04-02)<br>fig. 4D | 1-14 |
| A | JP 2019-152655 A (ZHOU, Yuchen) 12 September 2019 (2019-09-12) | 1-14 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 September 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/027987**

| Patent document cited in search report | | | Publication date (day/month/year) | Patent family member(s) | | | Publication date (day/month/year) |
|---|---|---|---|---|---|---|---|
| JP | 2013-238420 | A | 28 November 2013 | (Family: none) | | | |
| JP | 2004-535292 | A | 25 November 2004 | US claim 1, fig. 3 | 2004/0108253 | A1 | |
| | | | | WO | 2003/002260 | A1 | |
| | | | | FR | 2826592 | A1 | |
| WO | 2020/061696 | A1 | 02 April 2020 | JP fig. 4D | 2022-502638 | A | |
| | | | | US | 2021/0403856 | A1 | |
| | | | | CN | 112752847 | A | |
| | | | | KR | 10-2021-0086630 | A | |
| JP | 2019-152655 | A | 12 September 2019 | US | 2019/0270084 | A1 | |
| | | | | EP | 3534155 | A2 | |
| | | | | CN | 110218648 | A | |

Form PCT/ISA/210 (patent family annex) (January 2015)

**EP 4 390 401 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011155489 A
- JP 2021135063 A

**Non-patent literature cited in the description**

- **MOTOKAWA.** High Magnetic Field and Material Science. *Material Japan,* 1998, vol. 37, 926-930